# FASCICULE DE BREVET EUROPEEN

(11) **EP 1 767 187 B1**
(45) Date de publication et mention de la délivrance du brevet: **05.03.2014**
(21) Numéro de dépôt: 06300974.0
(22) Date de dépôt: 22.09.2006
(51) Int. Cl.: A61K 8/894, A61Q 5/06, A61K 8/58

(54) **Composition cosmétique comprenant un composé organique du silicium, et procédé de mise en forme des cheveux**
Kosmetische Zusammensetzung enthaltend eine Organosiliziumvebindung und Verfahren zur Haarformung
Cosmetic composition comprising an organic silicium compound and process for shaping hair

(30) Priorité: 23.09.2005 FR 0552856
(43) Date de publication de la demande: 28.03.2007
(73) Titulaire: L'Oréal, 75008 Paris (FR)
(72) Inventeur: Rollat, Isabelle, 75017, Paris (FR); Benabdillah, Katarina, 95130 Le Plessis-Bouchard (FR); Lerda, Patrice, 92600 Asnieres (FR)
(74) Mandataire: Caillet, Isabelle

(56) Documents cités:
- EP-A- 0 464 835
- EP-A- 0 603 046
- US-A- 4 344 763
- US-A1- 2005 027 138

## Description

L'invention se rapporte à une composition cosmétique comprenant un composé organique du silicium, de préférence non hydrosoluble et soluble dans les milieux alcooliques ou hydroalcooliques. L'invention concerne encore l'utilisation pour le traitement des matières kératiniques, notamment des cheveux, d'une composition cosmétique comprenant un composé organique du silicium, de préférence non hydrosoluble et soluble dans les milieux alcooliques ou hydroalcooliques. L'invention se rapporte enfin à un procédé de mise en forme des cheveux comprenant l'application sur les cheveux de ladite composition.

Les consommateurs aux cheveux fins recherchent des effets coiffants durables apportant de la masse, du corps et du volume aux cheveux.

Les produits de coiffage permettent une mise en forme non permanente des cheveux et permettent d'obtenir ces effets coiffants. Ils s'utilisent sur cheveux mouillés ou secs avant la mise en forme à la main ou à l'aide d'une brosse ou d'un peigne. Ils contiennent généralement un ou plusieurs actifs cosmétiques, tels que des polymères fixants, des épaississants, du glycérol, des silicones ou des cires par exemple.

Après leur application sur les cheveux et après séchage, ces produits durcissent de façon importante. Cela se traduit par un toucher corporisé et sec nécessaire au maintien et au volume de la coiffure.

Cependant, les produits de coiffage présentent l'inconvénient que les effets coiffants disparaissent au premier shampooing suivant l'application du produit de coiffage. Il faut donc les appliquer quotidiennement.

Il existe donc un besoin de disposer de produits de coiffage qui permettent d'obtenir ces effets coiffants, notamment d'apporter de la masse, du corps et du volume, mais qui soient résistants à plusieurs shampooings.

On connaît ainsi des documents FR 2 783 164, FR-2 783 167, FR 2 783 165 et FR 2 798 063 l'utilisation de certains composés organiques du silicium choisis parmi les silanes comprenant un atome de silicium et les siloxanes comprenant deux ou trois atomes de silicium, ces composés étant hydrosolubles, pour le maintien de la coiffure où la mise en forme des cheveux. Ces composés permettent d'obtenir des effets coiffants, par exemple de la masse, du corps, la facilité de démêlage, le dessin de boucle, résistant à plusieurs shampooings. Néanmoins, ces effets sont relativement faibles et irréguliers. Ils s'estompent après environ deux semaines.

On connaît par ailleurs du document WO 92/17184 l'utilisation de composés polysiloxanes comme agents pontants qui réagissent avec la surface de la peau pour former ainsi des complexes polysiloxanes permettant de lier un agent bioactif à la surface de la peau.

On connaît encore du document DE 4035033 des composés complexes polymères sphériques métalliques dont le ligand est constitué par un condensat de copolymère d'organosiloxane.

Par ailleurs, on recherche aussi pour la peau, notamment du visage humain, et les phanères, autres que les cheveux, en particulier les cils et les sourcils, des produits ayant des propriétés de soin, de protection et/ou de conditionnement. De tels produits peuvent être des fonds de teint, des rouges à lèvres, des crèmes ou des gels pour le corps ou le visage, des ombres à paupières, des eye-liners, des mascaras, des blush.

En outre, que se soit pour la chevelure ou la peau ou les lèvres, on recherche encore des compositions cosmétiques aptes à former sur ces matières kératiniques un film souple, qui suive les mouvements de la peau ou de la chevelure, sans effet de tiraillement, de lourdeur, ou de sensation rigide.

Le Demandeur a remarqué que l'application sur les cheveux de certains composés organiques du silicium, de préférence non hydrosolubles, et solubles dans les milieux alcooliques ou hydroalcooliques, permet d'obtenir des effets coiffants puissants et résistants à plusieurs shampooings. Ces composés contiennent des fonctions hydrolysables et polymérisables. Après application de ces composés sur les cheveux, puis mise en forme des cheveux, ces composés sèchent et polymérisent, formant des matériaux insolubles dans l'eau et dans le shampooing, permettant d'obtenir ainsi des effets coiffants durables.

L'invention a donc pour objet une composition cosmétique comprenant, dans un milieu cosmétiquement acceptable, au moins un composé organique du silicium de formule (1) :

(R₁O)ₓ(R₂)_{y}Si -(A)ₚ- [N R₃ - (A')_{p'}]_{q} - [N R'₃ - (A")_{p"}]_{q}' - Si - (R'₂)_{y'}(OR'₁)_{x'} (1)

où
R₁, R₂, R'₁ et R'₂ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x est un entier variant de 1 à 3,
y = 3-x,
x' est un entier variant de 1 à 3,
y' = 3-x',
p = 0 ou 1,
p' = 0 ou 1,
p" = 0 ou 1,
q = 0 ou 1,
q' = 0 ou 1,
étant entendu que au moins q ou q' est différent de zéro,
A, A' et A" représentent chacun indépendamment un radical divalent alkylène linéaire ou ramifié en C₁-C₂₀,
R₃ et R'₃ représentent chacun indépendamment un atome d'hydrogène ou une chaîne hydrocarbonée saturée ou insaturée, linéaire ou ramifiée contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₃-C₂₀, amine, amide, carboxyle, alcoxysilane, hydroxyle, carbonyle ou acyle,
ladite composition cosmétique comprenant en outre au moins un agent cosmétique choisi parmi les polymères de poids moléculaire au moins égal à 500, les agents tensioactifs et les corps gras non siliconés.

De préférence, ledit milieu cosmétiquement acceptable contient au moins un solvant alcoolique en C₁-C₈ et de 0 à 50% en poids d'eau par rapport au poids total de la composition.

Comme expliqué précédemment, R₁, R₂, R'₁ et R'₂ représentent chacun indépendamment une chaîne hydrocarbonée. Par chaîne hydrocarbonée, on entend de préférence une chaîne comportant de 1 à 10 atomes de carbone.

De même, R₃ et R'₃ peuvent représenter une chaîne hydrocarbonée. Dans ce cas, on entend de préférence une chaîne comportant de 1 à 10 atomes de carbone.

De préférence, le cycle aromatique comporte de 6 à 30 atomes de carbone. Encore plus préférentiellement, il désigne un radical phényle éventuellement substitué.

Selon un mode de réalisation particulier, le ou les composés organiques du silicium utilisés dans la composition selon l'invention présentent les caractéristiques suivantes :
R₁ = R'₁,
R₂ = R'₂,
x = x',
y = y',
p = p',
A = A',
q = 1, et
q'= 0.

Le ou les composés organiques du silicium utilisés dans la composition selon l'invention peuvent également présenter les caractéristiques suivantes, prises seules ou en combinaison :
- R₁, R₂, R'₁ et R'₂, identiques ou différents, représentent un alkyle en C₁-C₄,
- p = p' = 1,
- A et A', identiques ou différents, représentent un alkylène linéaire en C₁-C₄,
- R₃ est l'hydrogène.

De préférence, le composé de formule (1) est non hydrosoluble et soluble dans les milieux alcooliques ou hydroalcooliques.

Par composé soluble, on entend au sens de la présente invention un composé formant à la concentration de 1% dans le milieu de référence une solution microscopiquement isotrope et transparente.

Dans le cas contraire, le composé est dit insoluble dans le milieu correspondant.

De façon particulièrement préférée, le ou les composés organiques du silicium utilisés dans la composition selon l'invention sont choisis parmi les composés suivants :
- la bis[3-(triéthoxysilyl)propyl]amine, de formule :

   (CH₃CH₂O)₃ - Si - (CH₂)₃ - NH - (CH₂)₃ - Si - (OCH₂CH₃)₃,

   proposée par la société FLUOROCHEM,
- la bis[triméthoxysilylpropyl]amine, de formule :

   (CH₃O)₃- Si - (CH₂)₃- NH - (CH₂)₃- Si - (OCH₃)₃,

   proposée par la société GELEST,
- la bis[méthyldiéthoxysilylpropyl]amine, de formule :

   (CH₃CH₂O)₂CH₃- Si - (CH₂)₃- NH - (CH₂)₃- Si - CH₃(OCH₂CH₃)₂,

   proposée par la société GELEST, et
- la bis[(3-triméthoxysilyl)propyl]-éthylènediamine, de formule :

   (CH₃O)₃- Si - (CH₂)₃- NH -(CH₂)₂- NH- (CH₂)₃- Si - (OCH₃)₃,

   proposée par la société GELEST,

Un composé organique du silicium particulièrement préféré est la bis[3-(triéthoxysilyl)propyl]amine.

Le ou les composés organiques du silicium présents dans la composition selon l'invention peuvent représenter entre 0,5 et 50% en poids du poids total de la composition, de préférence de 2 à 25%.

La composition selon l'invention comprend au moins un agent cosmétique choisi parmi les polymères, les agents tensioactifs et les corps gras non siliconés.

Le ou les polymères pouvant être compris dans la composition selon l'invention peuvent être anioniques, cationiques, non ioniques ou amphotères et peuvent être siliconés ou non. Leur poids moléculaire est d'au moins 500.

Comme polymères anioniques, cationiques, non ioniques ou amphotères, on peut utiliser tous les polymères classiquement utilisés dans les compositions cosmétiques.

On peut citer par exemple les polymères siliconés non ioniques linéaires, modifiés ou non, tels que polydiméthylsiloxanes, les polydiméthylsiloxanes oxyéthylénés et/ou oxypropylénés.

On peut encore citer les silicones cationiques telles que les polydiméthylsiloxanes aminés.

Comme polymères siliconés anioniques, on peut citer le VS 80 (commercialisé par la société 3M), le LUVISET SI PUR (commercialisé par la société BASF).

Comme polymères non siliconés amphotères, on peut citer l'AMPHOMER LV 71 (commercialisé par la société NATIONAL STARCH) et le MERQUAT 280 (commercialisé par la société PALSON).

Comme polymères non siliconés non ioniques, on peut citer la polyvinylpyrrolidone (PVP) et les copolymères PVP/VA.

Comme polymères non siliconés anioniques, on peut citer le FIXATE G 100L (commercialisé par la société NOVEON), l'ULTRA HOLDSTRONG (commercialisé par la société BASF), le MEXOMERE PW (commercialisé par la société CHIMEX).

Comme polymères non siliconés cationiques, on peut citer le MERQUAT 100 (commercialisé par la société CALGON), le MERQUAT 550 (commercialisé par la société CALGON), le SALCARE SC 95 (commercialisé par la société CIBA), le SALCARE SC 96 (commercialisé par la société CIBA), le JAGUAR C13S (commercialisé par la société RHODIA), le JR 400 (commercialisé par la société AMERCHOL), le GAFQUAT 734 (commercialisé par la société ISP), le GAPQUAT 755 (commercialisé par la société ISP), le MEXOMERE PO (commercialisé par la société CHIMEX), le LUVIQUAT F905 (commercialisé par la société BASF).

Le ou les polymères pouvant être compris dans la composition selon l'invention peuvent également être choisis parmi les épaississants polymériques. Les épaississants polymériques utilisables dans la composition selon l'invention peuvent être choisis parmi tous les épaississants polymériques classiquement utilisés dans les compositions cosmétiques dont on veut augmenter la viscosité. On peut citer en particulier les hydroxyl éthyl ou propyl cellulose ou les acides polyacryliques. Parmi les épaississants associatifs, on peut citer le CARBOPOL 1382 (commercialisé par la société NOVEON), le PEMULEN TR1 (commercialisé par la société NOVEON), l'ACULYN 44 (commercialisé par la société ROHM et HAAS), l'ACULYN 46 (commercialisé par la société ROHM et HAAS), l'ACULYN 22 (commercialisé par la société ROHM et HAAS), l'ACULYN 28 (commercialisé par la société ROHM et HAAS), le VISCOPHOBE DB 1000 (commercialisé par la société DOW CHEMICAL).

Le ou les tensioactifs pouvant être compris dans la composition selon l'invention peuvent être choisis parmi tous les tensioactifs non-ioniques, anioniques, cationiques ou amphotères ou zwittérioniques classiquement utilisés dans les compositions cosmétiques. Parmi ceux-ci, on peut citer les alkyl sulfates, les alkyl benzènesulfates, les alkyl éthersulfates, les alkyl sulfonates, les sels d'ammonium quaternaire, les alkyl bétaïnes, les alkyl amidoalkyl bétaïnes, les alkyl phénols oxyalkylénés, les acides gras oxyalkylénés (alkyléther carboxylates), les alcools gras oxyalkylénés, les amides grasses oxyalkylénés, les esters d'acides gras oxyéthylénés ainsi que d'autres tensioactifs non-ioniques du type alkyl polyglycosides. Ces tensioactifs ont de préférence une HLB supérieure à 2.

Le ou les corps gras non siliconés pouvant être compris dans la composition selon l'invention peuvent être choisis parmi les alcools gras, les acides gras et leurs sels, les éthers gras, les esters gras, les amides gras comportant au moins une chaîne grasse hydrocarbonée saturée ou insaturée, linéaire ou ramifiée, comprenant de 10 à 30 atomes de carbone. Ces corps peuvent être des huiles, des cires, des beurres.

Parmi les alcools gras, on peut citer l'alcool cétylique, l'alcool cétylstéarylique, l'alcool stéarylique, l'alcool isostéarylique, l'alcool oléique, l'alcool béhénylique.

Parmi les éthers gras, on peut citer le distéréaryléther.

Parmi les esters gras, on peut citer le myristate d'isopropyle, le palmitate d'isopropyle, le néopentanoate d'isostéaryle.

Parmi les amides gras, on peut citer le diéthanolamide laurique, le N-oléoldihydrosphingosine.

Le ou les agents cosmétiques choisis parmi les polymères, les tensioactifs et les corps gras non siliconés représentent généralement, de 0,05 à 40%, de préférence de 0,1 à 20% et encore plus préférentiellement de 0,5 à 10%, en poids du poids total de la composition cosmétique.

Comme expliqué précédemment, la composition cosmétique contient un milieu cosmétiquement acceptable contenant de préférence au moins un solvant alcoolique en C₁-C₈ et de 0 à 50% en poids d'eau par rapport au poids total de la composition. Le ou les composés organiques du silicium présents dans la composition sont solubles dans ce milieu.

Le ou les solvants alcooliques en C₁-C₈ peuvent être choisis parmi tous types de solvants alcooliques classiquement utilisés dans les compositions cosmétiques. De préférence, le ou les solvants alcooliques sont choisis parmi les alcanols et les alcanediols.

A titre d'alcanols, on peut citer en particulier l'éthanol, le propanol et l'isopropanol, de préférence l'éthanol.

A titre d'alcanediols, on peut citer le propylène glycol et le pentanediol.

Le ou les solvants alcooliques représentent généralement de 40 à 95% en poids par rapport au poids total de la composition.

De préférence, le ou les solvants alcooliques représentent de 50 à 90% en poids par rapport au poids total de la composition.

Outre le ou les composés organiques du silicium, le ou les composés choisis parmi les polymères, les agents tensioactifs et les corps gras non siliconés, et le ou les éventuels solvants alcooliques et éventuellement l'eau, la composition cosmétique selon l'invention peut encore comprendre au moins un actif cosmétique additionnel choisi parmi les hydrolysats de protéines, les agents de gonflement, les agents propénétrants, les agents antichute, les agents antipelliculaires, les épaississants naturels ou synthétiques non polymériques, les agents de suspension, les agents réducteurs, les agents séquestrants, les agents opacifiants, les colorants, les filtres solaires, les vitamines ou provitamines, les parfums et les conservateurs, les ajusteurs de pH et leurs mélanges.

Les ajusteurs de pH peuvent être choisis parmi les agents alcalins, tels que, par exemple, l'ammoniaque, la monoéthanolamine, la diéthanolamine, la triéthanolamine, la 1,3-propanediamine, un hydroxyde alcalin, tel que le 2-amino-2-méthyl-1-propanol, ou bien parmi les agents acidifiants tel que par exemple l'acide phosphorique ou l'acide chlorhydrique.

Les colorants peuvent être choisis parmi les colorants directs ou les colorants d'oxydation.

Les colorants d'oxydation sont des composés ou mélanges de composés qui, en présence d'un oxydant, par exemple l'oxygène de l'air ou l'eau oxygénée, s'oxydent pour donner un composé ou un mélange de composés colorés.

Les colorants d'oxydation sont généralement choisis parmi les bases d'oxydation, les coupleurs et leurs mélanges.

A titre de colorants d'oxydation utilisables dans la composition selon l'invention, on peut citer par exemple la p-phénylène diamine, la p-toluylène diamine, le para-aminophénol et le 2-méthyl, 5-aminophénol, le méta-aminophénol, la résorcine.

Les colorants directs peuvent notamment être choisis parmi les colorants nitrés, azoïques, méthiniques ou anthraquinoniques, neutres, cationiques ou anioniques. On peut citer par exemple le Basic Red 1, le Basic Red 3, le Basic Red 22, le Basic Red 46, le Basic Red 51, le Basic Orange 31 et le Basic Yellow 87.

Les agents réducteurs peuvent être choisis parmi tous types d'agents réducteurs classiquement utilisés en cosmétique. On peut citer par exemple les thiols, les dithiols, les sulfites, les bisulfites, les (C₁-C₈) alkyl-phosphines, les réductones dont l'acide ascorbique et l'acide érythorbique.

Les agents séquestrants peuvent être choisis parmi tous les types d'agents séquestrants classiquement utilisés dans les compositions cosmétiques. On peut citer par exemple l'acide diéthylènetriamine pentaméthylène phosphonique, l'acide diéthylènetriamine tétraméthylène phosphonique, l'acide éthylènediamine tétraacétique, et leurs sels, notamment de sodium ou de potassium.

La composition selon l'invention peut également contenir un ou plusieurs autres acides organiques.

Les acides organiques additionnels sont généralement choisis parmi les acides comportant une ou plusieurs fonctions acide carboxylique, sulfonique, phosphonique ou phosphorique. Ils peuvent contenir d'autres fonctions chimiques, en particulier des fonctions hydroxy ou amino. Ils peuvent être saturés ou insaturés.

On peut citer en particulier l'acide acétique, l'acide propanoïque, l'acide butanoïque, l'acide lactique, l'acide glycolique, l'acide ascorbique, l'acide maléique, l'acide phtalique, l'acide succinique, la taurine, l'acide tartrique, l'acide gluconique, l'acide glucuronique et l'acide citrique.

La présence d'un ou plusieurs acides organiques dans la composition selon l'invention permet en particulier d'augmenter la proportion d'eau dans la composition.

La composition selon l'invention peut se présenter sous toutes les formes possibles pour une application sur les cheveux, notamment sous forme d'une solution du type lotion ou sérum, sous forme de gel, sous forme d'émulsion eau-dans-huile, huile-dans-eau ou multiple, de consistance liquide plus ou moins épaisse, telle que des laits et des crèmes plus ou moins onctueuses, ou des mousses.

La composition selon l'invention peut être en particulier un produit capillaire tel qu'un produit de coiffage tels qu'une lotion, un gel, une mousse ou une crème de coiffage, une crème de soin, un shampooing, un après-shampooing, ou une composition de coloration.

La composition selon l'invention peut être conditionnée sous différentes formes : des tubes, des pots mais aussi des vaporisateurs, des flacons pompe ou dans des récipients aérosol, afin d'assurer une application de la composition sous forme vaporisée ou sous forme de mousse.

Lorsque la composition est conditionnée sous forme d'aérosol, l'aérosol peut être bi-compartimenté.

Lorsque la composition est conditionnée dans un dispositif aérosol, elle comprend au moins un agent propulseur, qui peut être choisi parmi les hydrocarbures volatiles, tels que le n-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise le diméthyl éther.

Avantageusement, l'agent propulseur est présent à une concentration comprise entre 5 et 90% en poids par rapport au poids total de la composition dans le dispositif aérosol, et, plus particulièrement, à une concentration comprise entre 10 et 60%.

L'invention concerne encore l'utilisation pour le traitement des cheveux d'une composition comprenant, dans un milieu cosmétiquement acceptable, au moins un composé organique du silicium et de formule (1) :

(R₁O)ₓ(R₂)_{y}Si-(A)ₚ - [N R₃ - (A')_{p'}]_{q} - [N R'₃ - (A")_{p"}]_{q'} - Si - (R'₂)_{y'}(OR'₁)_{x'} (1)

où
R₁, R₂, R'₁ et R'₂ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x est un entier variant de 1 à 3,
y = 3-x,
x' est un entier variant de 1 à 3,
y' = 3-x',
p = 0 ou 1,
p' = 0 ou 1,
p" = 0 ou 1,
q = 0 ou 1,
q' = 0 ou 1,
étant entendu que au moins q ou q' est différent de zéro,
A, A' et A" représentent chacun indépendamment un radical divalent alkylène linéaire ou ramifié en C₁-C₂₀,
R₃ et R'₃ représentent chacun indépendamment un atome d'hydrogène ou une chaîne hydrocarbonée saturée ou insaturée, linéaire ou ramifiée contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₃-C₂₀, amine, amide, carboxyle, alcoxysilane, hydroxyle, carbonyle ou acyle.

De préférence, ledit milieu cosmétiquement acceptable contient au moins un solvant alcoolique en C₁-C₈ et de 0 à 50 % en poids d'eau par rapport au poids total de la composition.

De préférence, le composé de formule (1), est non hydrosoluble et soluble dans les milieux alcooliques ou hydroalcooliques.

Les modes de réalisation préférés décrits précédemment et concernant le ou les composés organiques du silicium, le ou les solvants alcooliques, la présence éventuelle d'actifs cosmétiques et d'acides organiques, la forme de la composition et son conditionnement sont applicables à la composition utilisée selon l'invention.

La composition utilisée selon l'invention peut être utilisée pour le soin, la protection ou le maquillage des matières kératiniques humaines telles que la peau, les cheveux, les cils, les ongles ou les lèvres.

De préférence, le traitement selon l'invention est une mise en forme.

L'invention concerne enfin un procédé de mise en forme des cheveux, mettant en oeuvre la composition utilisée selon l'invention.

Dans le procédé selon l'invention, la composition utilisée selon l'invention et comprenant le ou les composés organiques du silicium est appliquée sur les cheveux, rincés ou non. L'application se fait de préférence sous la forme d'un spray, soit à l'aide d'un flacon pompe, soit à l'aide d'un aérosol.

La composition utilisée selon l'invention est ensuite laissée à poser sur les cheveux, pour lui permettre de pénétrer dans le cheveu, en général pendant au plus 30 minutes, de préférence pendant 5 à 15 minutes.

Les cheveux peuvent être rincés à l'eau après l'application de la composition et le temps de pose.

Les cheveux sont alors de préférence mis en forme, par exemple par brushing, par application de pinces plates.

De préférence, les cheveux sont séchés au sèche cheveux et mis en forme par brushing.

Lors du séchage des cheveux, le ou les composés du silicium, qui sont des composés monomères, sèchent et polymérisent, formant des matériaux insolubles dans l'eau et dans le shampooing.

Les cheveux ayant été mis en forme par le procédé selon l'invention présentent plus de volume, de masse, de texture, et ils se mettent mieux en forme.

L'effet coiffant obtenu est supérieur à celui obtenu lors de la mise en forme des cheveux sans application de la composition selon l'invention.

Les cheveux peuvent être lavés par shampooings puis remis en forme par brushing, les même effets coiffants sont obtenus. Les effets sont résistants à environ 10 shampooings.

La présente invention est illustrée par les exemples suivants.

### Exemples

Les pourcentages qui suivent sont exprimés en poids par rapport au poids de matière active.

### Exemple 1

On formule une composition cosmétique selon l'invention, sous forme de lotion, de composition suivante :

| | |
|---|---|
| bis[3-(triéthoxysilyl)propyl]amine | 10% |
| PDMS oxyéthyléné oxypropyléné (DC Q 2-5220 de Dow Corning) | 1% |
| Ethanol | 89% |

### Exemple 2

On formule une composition cosmétique selon l'invention, sous forme de gel, de composition suivante :

| | |
|---|---|
| bis[3-(triéthoxysilyl)propyl]amine | 10% |
| hydroxypropyl cellulose (Klucel G de Aqualon - Hercules) | 2% |
| Ethanol | 78% |
| Eau | 10% |

### Exemple 3

On formule une composition cosmétique selon l'invention, sous forme d'une mousse de coiffage, de composition suivante :

| | |
|---|---|
| bis[3-(triéthoxysilyl)propyl]amine | 10% |
| monolaurate de sorbitane oxyéthyléné (20 OE) | 5% |
| Ethanol | 65% |
| Eau | 15% |
| isobutane/propane (85/15) | 5% |

### Exemple 4

On formule une composition cosmétique selon l'invention, sous forme d'une crème de coiffage, de composition suivante :

| | |
|---|---|
| bis[3-(triéthoxysilyl)propyl]amine | 10% |
| Alcool cétylstéarylique | 5% |
| hydroxypropyl cellulose (Klucel G de Aqualon - Hercules) | 1 % |
| Ethanol | 74% |
| Eau | 10% |

### Exemple 5

On prépare une composition cosmétique de coloration. Pour cela, on formule d'une part une composition A selon l'invention, comprenant des colorants d'oxydation, et d'autre part une composition B comprenant de l'eau oxygénée.

### Composition A

| | |
|---|---|
| bis[3-(triéthoxysilyl)propyl]amine | 2% |
| p-phénylène diamine | 0,25% |
| 2-méthyl 5-aminophénol | 0,35% |
| agent séquestrant | qs |
| agent réducteur | qs |
| solution aqueuse d'ammoniac à 20% | 10% |
| Ethanol | 50% |
| Décyl polyglucoside | 2,5% |
| Eau | qsp 100% |

### Composition B

### Eau oxygénée 20 volumes

Les compositions A et B se mélangent poids par poids avant d'être appliquée sur les cheveux.

### Exemple 6

On formule une composition cosmétique de coloration selon l'invention, de composition suivante :

| | |
|---|---|
| bis[3-(triéthoxysilyl)propyl]amine | 3% |
| Décyl polyglucoside | 2,5% |
| Basic Red 51 | 0,2 % |
| Agent de pH | qs pH=8 |
| Ethanol | 50% |
| Eau | qsp 100% |

Lorsque les compositions des exemples 1 à 6 sont appliquées sur les cheveux, on obtient des effets coiffants durables, résistants à environ 10 shampooings.

## Revendications

1. Composition cosmétique comprenant, dans un milieu cosmétiquement acceptable au moins un composé organique du silicium de formule (1) :
(R₁O)ₓ(R₂)_{y}Si-(A)ₚ - [N R₃ - (A')_{p'}]_{q} - [NR'₃ - (A")_{p"}]_{q'} - Si - (R'₂)_{y'}(OR'₁)_{x'} (1)
où
R₁, R₂, R'₁ et R'₂ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x est un entier variant de 1 à 3,
y = 3-x,
x' est un entier variant de 1 à 3,
y' = 3-x',
p = 0 ou 1,
p' = 0 ou 1,
p"=0 ou 1,
q = 0 ou 1,
q' =0 ou 1,
étant entendu que au moins q ou q' est différent de zéro, A, A' et A" représentent chacun indépendamment un radical divalent alkylène linéaire ou ramifié en C₁-C₂₀,
R₃ et R'₃ représentent chacun indépendamment un atome d'hydrogène ou une chaîne hydrocarbonée saturée ou insaturée, linéaire ou ramifiée contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₃-C₂₀, amine, amide, carboxyle, alcoxysilane, hydroxyle, carbonyle ou acyle,
ladite composition cosmétique comprenant en outre au moins un agent cosmétique choisi parmi les polymères de poids moléculaire au moins égal à 500, les agents tensioactifs et les corps gras non siliconés.

2. Composition selon la revendication 1 **caractérisée en ce que** ledit milieu cosmétiquement acceptable contient au moins un solvant alcoolique en C₁-C₈ et de 0 à 50% en poids d'eau par rapport au poids total de la composition.

3. Composition selon l'une quelconque des revendications précédentes **caractérisée en ce que** :
R₁ = R'_{1.}
R₂ = R'_{2,}
x = x',
y = y',
p = p',
A = A',
q = 1, et
q' = 0.

4. Composition selon l'une quelconque des revendications précédentes **caractérisée en ce que** R₁, R₂, R'₁ et R'₂, identiques ou différents, représentent un alkyle en C₁-C₄.

5. Composition selon l'une quelconque des revendications précédentes **caractérisée en ce que** p = p' = 1.

6. Composition selon l'une quelconque des revendications précédentes **caractérisée en ce que** A et A', identiques ou différents, représentent un alkylène linéaire en C₁-C₄.

7. Composition selon l'une quelconque des revendications précédentes **caractérisée en ce que** R₃ est l'hydrogène.

8. Composition selon l'une quelconque des revendications 1 à 7, **caractérisée en ce que** le composé de formule (1) est non hydrosoluble et soluble dans les milieux alcooliques et/ou hydroalcooliques.

9. Composition selon l'une quelconque des revendications précédentes **caractérisée en ce que** le ou les composés organiques du silicium sont choisis parmi :
- la bis[3-(triéthoxysilyl)propyl]amine, de formule :
(CH₃CH₂O)₃- Si - (CH₂)₃- NH - (CH₂)₃- Si - (OCH₂CH₃)₃,
- la bis[triméthoxysilylpropyl]amine, de formule :
(CH₃O)₃- Si - (CH₂)₃- NH - (CH₂)₃- Si - (OCH₃)₃,
- la bis[méthyldiéthoxysilylpropyl]amine, de formule :
(CH₃CH₂O)₂CH₃- Si - (CH₂)₃- NH - (CH₂)₃- Si - CH₃(OCH₂CH₃)₂,
- la bis[(3-triméthoxysilyl)propyl]-éthylènediamine, de formule :
(CH₃O)₃- Si - (CH₂)₃- NH -(CH₂)₂- NH- (CH₂)₃- Si - (OCH₃)₃, et

10. Composition cosmétique selon la revendication 9 **caractérisée en ce que** le composé organique du silicium est la bis[3-(triéthoxysilyl)propyl]amine.

11. Composition selon l'une quelconque des revendications précédentes **caractérisée en ce que** le ou les composés organiques du silicium représentent de 0,5 à 50%, de préférence de 2 à 25%, en poids par rapport au poids total de la composition.

12. Composition selon l'une quelconque des revendications 1 à 11 **caractérisée en ce que** le ou agents cosmétiques choisis parmi les polymères, les tensioactifs et les corps gras non siliconés représentent de 0,05 à 40%, de préférence de 0,1 à 20% et encore plus préférentiellement de 0,5% à 10%, en poids du poids total de la composition.

13. Composition selon l'une quelconque des revendications 2 à 12 **caractérisée en ce que** le ou les solvants alcooliques en C₁-C₈ sont choisis parmi les alcanols et les alcanediols.

14. Composition selon la revendication 13 **caractérisée en ce que** le ou les solvants sont choisis parmi l'éthanol, le propanol, l'isopropanol, de préférence l'éthanol.

15. Composition cosmétique selon l'une quelconque des revendications 2 à 14 **caractérisée en ce que** le ou les solvants alcooliques représentent de 40 à 95%, de préférence de 50 à 90% en poids par rapport au poids total de la composition.

16. Composition selon l'une quelconque des revendications précédentes **caractérisée en ce qu'**elle comprend en outre au moins un actif cosmétique additionnel choisi parmi les hydrolysats de protéines, les agents de gonflement et de pénétration, les agents antichute, les agents antipelliculaires, les épaississants naturels ou synthétiques non polymériques, les agents de suspension, les agents séquestrants, les agents réducteurs, les agents opacifiants, les colorants, les filtres solaires, les vitamines ou provitamines, les parfums et les conservateurs, les ajusteurs de pH et leurs mélanges.

17. Composition selon l'une quelconque des revendications précédentes **caractérisée en ce qu'**elle comprend un ou plusieurs acides organiques.

18. Composition selon la revendication 17 **caractérisée en ce que** le ou les acides organiques sont choisis parmi l'acide acétique, l'acide propanoïque, l'acide butanoïque, l'acide lactique, l'acide glycolique, l'acide ascorbique, l'acide maléique, l'acide phtalique, l'acide succinique, la taurine, l'acide tartrique, l'acide gluconique, l'acide glucuronique et l'acide citrique.

19. Utilisation pour le traitement des cheveux d'une composition cosmétique comprenant, dans un milieu cosmétiquement acceptable, au moins un composé organique du silicium de formule (1) :
(R₁O)ₓ(R₂)_{y}Si- (A)ₚ- [N R₃ - (A')_{p'}]_{q} - [N R'₃ - (A")_{p"}]_{q'} - Si - (R'₂)_{y'}(OR'₁)_{x'} (1)
où
R₁, R₂, R'₁ et R'₂ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x est un entier variant de 1 à 3,
y = 3-x,
x' est un entier variant de 1 à 3,
y' = 3-x',
p = 0 ou 1,
p' = 0 ou 1,
p" = 0 ou 1,
q = 0 ou 1,
q' = 0 ou 1,
étant entendu que au moins q ou q' est différent de zéro, A, A' et A" représentent chacun indépendamment un radical divalent alkylène linéaire ou ramifié en C₁-C₂₀,
R₃ et R'₃ représentent chacun indépendamment un atome d'hydrogène ou une chaîne hydrocarbonée saturée ou insaturée, linéaire ou ramifiée contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₃-C₂₀, amine, amide, carboxyle, alcoxysilane, hydroxyle, carbonyle ou acyle.

20. Utilisation selon la revendication 19, **caractérisée en ce que** ledit milieu cosmétiquement acceptable contient au moins un solvant alcoolique en C₁-C₈ et de 0 à 50% en poids d'eau par rapport au poids total de la composition.

21. Utilisation selon l'une quelconque des revendications 19 ou 20, **caractérisée en ce que** le composé de formule (1) est non hydrosoluble et soluble dans les milieux alcooliques et/ou hydroalcooliques.

22. Utilisation selon l'une quelconque des revendications 19 à 21, **caractérisée en ce que** le traitement est une mise en forme des cheveux.

23. Utilisation selon la revendication 19 à 22 **caractérisée en ce que** :
R₁ = R'₁,
R₂ = R'₂,
x = x',
y = y',
p = p',
A = A',
q = 1, et
q' = 0.

24. Utilisation selon l'une quelconque des revendications 19 à 23 **caractérisée en ce que** R₁, R₂, R'₁ et R'₂, identiques ou différents, représentent un alkyle en C₁-C₄.

25. Utilisation selon l'une quelconque des revendications 19 à 24 **caractérisée en ce que** p = p' = 1.

26. Utilisation selon l'une quelconque des revendications 19 à 25 **caractérisée en ce que** A et A', identiques ou différents, représentent un alkylène linéaire en C₁-C₄.

27. Utilisation selon l'une quelconque des revendications 19 à 26 **caractérisée en ce que** R₃ est l'hydrogène.

28. Utilisation selon l'une quelconque des revendications 19 à 27 **caractérisée en ce que** le ou les composés organiques du silicium sont choisis parmi :
- la bis[3-(triéthoxysilyl)propyl]amine, de formule :
(CH₃CH₂O)₃- Si - (CH₂)₃- NH - (CH₂)₃- Si - (OCH₂CH₃)₃,
- la bis[triméthoxysilylpropyl]amine, de formule :
(CH₃O)₃- Si - (CH₂)₃- NH - (CH₂)₃- Si - (OCH₃)₃,
- la bis[méthyldiéthoxysilylpropyl]amine, de formule :
(CH₃CH₂O)₂CH₃- Si - (CH₂)₃- NH - ((CH₂)₃ - Si - CH₃(OCH₂CH₃)₂,
- la bis[(3-triméthoxysilyl)propyl]-éthylènediamine, de formule :
(CH₃O)₃- Si - (CH₂)₃- NH -(CH₂)₂- NH- (CH₂)₃- Si - (OCH₃)₃, et

29. Utilisation cosmétique selon la revendication 28 **caractérisée en ce que** le composé organique du silicium est la bis[3-(triéthoxysilyl)propyl]amine.

30. Utilisation selon l'une quelconque des revendications 19 à 29 **caractérisée en ce que** le composé organique du silicium représente de 0,5 à 25%, de préférence de 2 à 15%, en poids par rapport au poids total de la composition.

31. Utilisation selon l'une quelconque des revendications 20 à 30 **caractérisée en ce que** le ou les solvants alcooliques en C₁-C₈ sont choisis parmi les alcanols et les alcanediols.

32. Utilisation selon la revendication 31 **caractérisée en ce que** le ou les solvants sont choisis parmi le méthanol, l'éthanol, le propanol, l'isopropanol, de préférence l'éthanol.

33. Utilisation selon l'une quelconque des revendications 20 à 32 **caractérisée en ce que** le ou les solvants alcooliques représentent de 40 à 95%, de préférence de 50 à 90% en poids par rapport au poids total de la composition.

34. Utilisation selon l'une quelconque des revendications 19 à 33 **caractérisée en ce que** la composition cosmétique contient au moins un agent cosmétique choisi parmi les polymères, les agents tensioactifs et les corps gras non siliconés.

35. Utilisation selon l'une quelconque des revendications 19 à 34 **caractérisée en ce que** la composition comprend un ou plusieurs acides organiques.

36. Utilisation selon la revendication 35 **caractérisée en ce que** le ou les acides organiques sont choisis parmi l'acide acétique, l'acide propanoïque, l'acide butanoïque, l'acide lactique, l'acide glycolique, l'acide ascorbique, l'acide maléique, l'acide phtalique, l'acide succinique, la taurine, l'acide tartrique, l'acide gluconique, l'acide glucuronique et l'acide citrique.

37. Procédé de mise en forme des cheveux **caractérisé en ce qu'**il comprend les étapes suivantes :
- une composition telle que définie dans l'une quelconque des revendications 1 à 36 est appliquée sur les cheveux,
- on laisse poser ladite composition, de préférence au plus 30 minutes, mieux entre 5 et 15 minutes,
- les cheveux sont mis en forme.

38. Procédé selon la revendication 37 **caractérisée en ce que** les cheveux sont rincés à l'eau après le temps de pose et avant la mise en forme.

## Patentansprüche

1. Kosmetische Zusammensetzung, die in einem kosmetisch unbedenklichen Medium mindestens eine organische Siliciumverbindung der Formel (1):
(R₁O)ₓ(R₂)_{y}Si- (A)ₚ- [NR₃- (A')_{p'}]_{q}- [NR'₃- (A")_{p"}]_{q'}-Si-(R'₂)_{y'} (OR'₁)_{x'} (1)
umfasst, wobei
R₁, R₂, R'₁ und R'₂ jeweils unabhängig für eine lineare oder verzweigte, gesättigte oder ungesättigte Kohlenwasserstoffkette, die gegebenenfalls ein oder mehrere Heteroatome enthält und gegebenenfalls durch eine oder mehrere Gruppen, die aus Ether-, Ester-, Amin-, Amid-, Carboxyl-, Hydroxyl- und Carbonylgruppen ausgewählt sind, unterbrochen oder substituiert ist, stehen,
x für eine ganze Zahl von 1 bis 3 steht,
y = 3-x,
x' für eine ganze Zahl von 1 bis 3 steht,
y' = 3-x',
p = 0 oder 1,
p' = 0 oder 1,
p" = 0 oder 1,
q = 0 oder 1,
q' = 0 oder 1,
mit der Maßgabe, dass mindestens q oder q' von Null verschieden ist,
A, A' und A" jeweils unabhängig für einen linearen oder verzweigten zweiwertigen C₁-C₂₀-Alkylenrest stehen,
R₃ und R'₃ jeweils unabhängig für ein Wasserstoffatom oder eine lineare oder verzweigte, gesättigte oder ungesättigte Kohlenwasserstoffkette, die gegebenenfalls ein oder mehrere Heteroatome enthält und gegebenenfalls durch eine oder mehrere Ether-, C₁-C₂₀-Alkoholester-, Amin-, Carboxyl-, Alkoxysilan-, C₆-C₃₀-Aryl-, Hydroxyl- oder Carbonylgruppen unterbrochen oder substituiert ist, oder einen gegebenenfalls heterocyclischen aromatischen Ring, der gegebenenfalls durch eine oder mehrere C₃-C₂₀-Alkoholester-, Amin-, Amid-, Carboxyl-, Alkoxysilan-, Hydroxyl-, Carbonyl- oder Acylgruppen substituiert ist, stehen,
wobei die kosmetische Zusammensetzung außerdem mindestens ein kosmetisches Mittel, das aus Polymeren mit einem Molekulargewicht von mindestens 500, Tensiden und Nichtsilikon-Fettsubstanzen ausgewählt ist, umfasst.

2. Zusammensetzung nach Anspruch 1, **dadurch gekennzeichnet, dass** das kosmetisch unbedenkliche Medium mindestens ein C₁-C₈-Alkohollösungsmittel und 0 bis 50 Gew.-% Wasser, bezogen auf das Gesamtgewicht der Zusammensetzung, enthält.

3. Zusammensetzung nach einem der vorhergehenden Ansprüche, **dadurch gekennzeichnet, dass**:
R₁ = R'₁,
R₂ = R'₂,
x = x',
y = y',
p = p',
A = A',
q = 1 und
q' = 0.

4. Zusammensetzung nach einem der vorhergehenden Ansprüche, **dadurch gekennzeichnet, dass** R₁, R₂, R'₁ und R'₂ gleich oder verschieden sind und für ein C₁-C₄-Alkyl stehen.

5. Zusammensetzung nach einem der vorhergehenden Ansprüche, **dadurch gekennzeichnet, dass** p = p' = 1.

6. Zusammensetzung nach einem der vorhergehenden Ansprüche, **dadurch gekennzeichnet, dass** A und A' gleich oder verschieden sind und für ein lineares C₁-C₄-Alkylen stehen.

7. Zusammensetzung nach einem der vorhergehenden Ansprüche, **dadurch gekennzeichnet, dass** R₃ Wasserstoff ist.

8. Zusammensetzung nach einem der Ansprüche 1 bis 7, **dadurch gekennzeichnet, dass** die Verbindung der Formel (1) wasserunlöslich und in alkoholischen und/oder wässrig-alkoholischen Medien löslich ist.

9. Zusammensetzung nach einem der vorhergehenden Ansprüche, **dadurch gekennzeichnet, dass** die organische Siliciumverbindung bzw. die organischen Siliciumverbindungen aus:
Bis[triethoxysilylpropyl]amin der Formel:
(CH₃CH₂O)₃-Si- (CH₂)₃-NH-(CH₂)₃-Si-(OCH₂CH₃)₃,
Bis[trimethoxysilylpropyl]amin der Formel:
(CH₃O)₃-Si- (CH₂)₃-NH- (CH₂)₃-Si- (OCH₃)₃,
Bis[methyldiethoxysilylpropyl]amin der Formel:
(CH₃CH₂O)₂CH₃-Si- (CH₂) ₃-NH- (CH₂) ₃-Si-CH₃ (OCH₂CH₃)₂,
Bis[3(-trimethoxysilyl)propyl]ethylendiamin der Formel:
(CH₃O)₃-Si- (CH₂)₃-NH- (CH₂)₂-NH- (CH₂)₃-Si- (OCH₃)₃
ausgewählt ist bzw. sind, und

10. Zusammensetzung nach Anspruch 9, **dadurch gekennzeichnet, dass** es sich bei der organischen Siliciumverbindung um Bis[3-(triethoxysilyl)-propyl]amin handelt.

11. Zusammensetzung nach einem der vorhergehenden Ansprüche, **dadurch gekennzeichnet, dass** die organische Siliciumverbindung bzw. die organischen Siliciumverbindungen 0,5 bis 50 Gew.-%, vorzugsweise 2 bis 25 Gew.-%, bezogen auf das Gesamtgewicht der Zusammensetzung, ausmacht bzw. ausmachen.

12. Zusammensetzung nach einem der Ansprüche 1 bis 11, **dadurch gekennzeichnet, dass** die aus Polymeren, Tensiden und Nichtsilikon-Fettsubstanzen ausgewählten kosmetischen Mittel 0,05 bis 40 Gew.-%, vorzugsweise 0,1 bis 20 Gew.-% und besser 0,5 bis 10 Gew.-%, bezogen auf das Gesamtgewicht der Zusammensetzung, ausmachen.

13. Zusammensetzung nach einem der Ansprüche 2 bis 12, **dadurch gekennzeichnet, dass** das C₁-C₈-Alkohollösungsmittel bzw. die C₁-C₈-Alkohollösungsmittel aus Alkanolen und Alkandiolen ausgewählt ist bzw. sind.

14. Zusammensetzung nach Anspruch 13, **dadurch gekennzeichnet, dass** das Lösungsmittel bzw. die Lösungsmittel aus Ethanol, Propanol, Isopropanol, vorzugsweise Ethanol, ausgewählt ist bzw. sind.

15. Kosmetische Zusammensetzung nach einem der Ansprüche 2 bis 14, **dadurch gekennzeichnet, dass** das Alkohollösungsmittel bzw. die Alkohollösungsmittel 40 bis 95 Gew.-%, vorzugsweise 50 bis 90 Gew.-%, bezogen auf das Gesamtgewicht der Zusammensetzung, ausmacht bzw. ausmachen.

16. Zusammensetzung nach einem der vorhergehenden Ansprüche, **dadurch gekennzeichnet, dass** sie außerdem mindestens einen zusätzlichen kosmetischen Wirkstoff umfasst, der aus Proteinhydrolysaten, Quellmitteln, Penetriermitteln, Mitteln zur Bekämpfung von Haarverlust, Antischuppenmitteln, natürlichen oder synthetischen nichtpolymeren Verdickern, Suspendiermitteln, Sequestriermitteln, Reduktionsmitteln, Trübungsmitteln, Farbmitteln, Lichtschutzmitteln, Vitaminen oder Provitaminen, Duftstoffen und Konservierungsmitteln, Mitteln zur Einstellung des pH-Werts und Mischungen davon ausgewählt ist.

17. Zusammensetzung nach einem der vorhergehenden Ansprüche, **dadurch gekennzeichnet, dass** sie eine oder mehrere organische Säuren umfasst.

18. Zusammensetzung nach Anspruch 17, **dadurch gekennzeichnet, dass** die organische Säure bzw. die organischen Säuren aus Essigsäure, Propansäure, Butansäure, Milchsäure, Glykolsäure, Ascorbinsäure, Maleinsäure, Phthalsäure, Bernsteinsäure, Taurin, Weinsäure, Gluconsäure, Glucuronsäure und Citronensäure ausgewählt ist bzw. sind.

19. Verwendung einer kosmetischen Zusammensetzung, die in einem kosmetisch unbedenklichen Medium mindestens eine organische Siliciumverbindung der Formel (1) :
(R₁O)ₓ(R₂)_{y}Si-(A)ₚ-[NR₃-(A')_{p'}]_{q}-[NR'₃-(A")_{p"}]_{q'}-Si-(R'₂)_{y'}(OR'₁)_{x'} (1)
umfasst, wobei
R₁, R₂, R'₁ und R'₂ jeweils unabhängig für eine lineare oder verzweigte, gesättigte oder ungesättigte Kohlenwasserstoffkette, die gegebenenfalls ein oder mehrere Heteroatome enthält und gegebenenfalls durch eine oder mehrere Gruppen, die aus Ether-, Ester-, Amin-, Amid-, Carboxyl-, Hydroxyl- und Carbonylgruppen ausgewählt sind, unterbrochen oder substituiert ist, stehen,
x für eine ganze Zahl von 1 bis 3 steht,
y = 3-x,
x' für eine ganze Zahl von 1 bis 3 steht,
y' = 3-x',
p = 0 oder 1,
p' = 0 oder 1,
p" = 0 oder 1,
q = 0 oder 1,
q' = 0 oder 1,
mit der Maßgabe, dass mindestens q oder q' von Null verschieden ist,
A, A' und A" jeweils unabhängig für einen linearen oder verzweigten zweiwertigen C₁-C₂₀-Alkylenrest stehen,
R₃ und R'₃ jeweils unabhängig für ein Wasserstoffatom oder eine lineare oder verzweigte, gesättigte oder ungesättigte Kohlenwasserstoffkette, die gegebenenfalls ein oder mehrere Heteroatome enthält und gegebenenfalls durch eine oder mehrere Ether-, C₁-C₂₀-Alkoholester-, Amin-, Carboxyl-, Alkoxysilan-, C₆-C₃₀-Aryl-, Hydroxyl- oder Carbonylgruppen unterbrochen oder substituiert ist, oder einen gegebenenfalls heterocyclischen aromatischen Ring, der gegebenenfalls durch eine oder mehrere C₃-C₂₀-Alkoholester-, Amin-, Amid-, Carboxyl-, Alkoxysilan-, Hydroxyl-, Carbonyl- oder Acylgruppen substituiert ist, stehen,
zur Behandlung von Haaren.

20. Verwendung nach Anspruch 19, **dadurch gekennzeichnet, dass** das kosmetisch unbedenkliche Medium mindestens ein C₁-C₈-Alkohollösungsmittel und 0 bis 50 Gew.-% Wasser, bezogen auf das Gesamtgewicht der Zusammensetzung, enthält.

21. Verwendung nach einem der Ansprüche 19 oder 20, **dadurch gekennzeichnet, dass** die Verbindung der Formel (1) wasserunlöslich und in alkoholischen und/oder wässrig-alkoholischen Medien löslich ist.

22. Verwendung nach einem der Ansprüche 19 bis 21, **dadurch gekennzeichnet, dass** es sich bei der Behandlung um eine Verformung von Haaren handelt.

23. Verwendung nach Anspruch 19 bis 22, **dadurch gekennzeichnet, dass**:
R₁ = R'₁,
R₂ = R'₂,
x = x',
y = y',
p = p',
A = A',
q = 1 und
q' = 0.

24. Verwendung nach einem der Ansprüche 19 bis 23, **dadurch gekennzeichnet, dass** R₁, R₂, R'₁ und R'₂ gleich oder verschieden sind und für ein C₁-C₄-Alkyl stehen.

25. Verwendung nach einem der Ansprüche 19 bis 24, **dadurch gekennzeichnet, dass** p = p' = 1.

26. Verwendung nach einem der Ansprüche 19 bis 25, **dadurch gekennzeichnet, dass** A und A' gleich oder verschieden sind und für ein lineares C₁-C₄-Alkylen stehen.

27. Verwendung nach einem der Ansprüche 19 bis 26, **dadurch gekennzeichnet, dass** R₃ Wasserstoff ist.

28. Verwendung nach einem der Ansprüche 19 bis 27, **dadurch gekennzeichnet, dass** die organische Siliciumverbindung bzw. die organischen Siliciumverbindungen aus:
Bis[3-(triethoxysilyl)propyl]amin der Formel:
(CH₃CH₂O)₃-Si-(CH₂)₃-NH-(CH₂)₃-Si-(OCH₂CH₃)₃,
Bis[trimethoxysilylpropyl]amin der Formel:
(CH₃O)₃-Si- (CH₂)₃-NH- (CH₂)₃-Si- (OCH₃)₃,
Bis[methyldiethoxysilylpropyl]amin der Formel:
(CH₃CH₂O) ₂CH₃-Si- (CH₂) ₃-NH- (CH₂) ₃-Si-CH₃ (OCH₂CH₃)₂,
Bis[3-(trimethoxysilyl)propyl]ethylendiamin der Formel:
(CH₃O)₃-Si- (CH₂)₃-NH- (CH₂)₂-NH- (CH₂)₃-Si- (OCH₃)₃
ausgewählt ist bzw. sind, und

29. Kosmetische Verwendung nach Anspruch 28, **dadurch gekennzeichnet, dass** es sich bei der organischen Siliciumverbindung um Bis[3-(triethoxysilyl)-propyl]amin handelt.

30. Verwendung nach einem der Ansprüche 19 bis 29, **dadurch gekennzeichnet, dass** die organische Siliciumverbindung 0,5 bis 25 Gew.-%, vorzugsweise 2 bis 15 Gew.-%, bezogen auf das Gesamtgewicht der Zusammensetzung, ausmacht.

31. Verwendung nach einem der Ansprüche 20 bis 30, **dadurch gekennzeichnet, dass** das C₁-C₈-Alkohollösungsmittel bzw. die C₁-C₈-Alkohollösungsmittel aus Alkanolen und Alkandiolen ausgewählt ist bzw. sind.

32. Verwendung nach Anspruch 31, **dadurch gekennzeichnet, dass** das Lösungsmittel bzw. die Lösungsmittel aus Methanol, Ethanol, Propanol, Isopropanol, vorzugsweise Ethanol, ausgewählt ist bzw. sind.

33. Verwendung nach einem der Ansprüche 20 bis 32, **dadurch gekennzeichnet, dass** das Alkohollösungsmittel bzw. die Alkohollösungsmittel 40 bis 95 Gew.-%, vorzugsweise 50 bis 90 Gew.-%, bezogen auf das Gesamtgewicht der Zusammensetzung, ausmacht bzw. ausmachen.

34. Verwendung nach einem der Ansprüche 19 bis 33, **dadurch gekennzeichnet, dass** die kosmetische Zusammensetzung mindestens ein kosmetisches Mittel, das aus Polymeren, Tensiden und Nichtsilikon-Fettsubstanzen ausgewählt ist, umfasst.

35. Verwendung nach einem der Ansprüche 19 bis 34, **dadurch gekennzeichnet, dass** die Zusammensetzung eine oder mehrere organische Säuren umfasst.

36. Verwendung nach Anspruch 35, **dadurch gekennzeichnet, dass** die organische Säure bzw. die organischen Säuren aus Essigsäure, Propansäure, Butansäure, Milchsäure, Glykolsäure, Ascorbinsäure, Maleinsäure, Phthalsäure, Bernsteinsäure, Taurin, Weinsäure, Gluconsäure, Glucuronsäure und Citronensäure ausgewählt ist bzw. sind.

37. Verfahren zur Verformung von Haaren, **dadurch gekennzeichnet, dass** es folgende Schritte umfasst:
- eine Zusammensetzung gemäß einem der Ansprüche 1 bis 36 wird auf die Haare aufgebracht,
- die Zusammensetzung wird einwirken gelassen, vorzugsweise höchstens 30 Minuten, besser zwischen 5 und 15 Minuten,
- die Haare werden verformt.

38. Verfahren nach Anspruch 37, **dadurch gekennzeichnet, dass** man die Haare nach der Einwirkzeit und vor dem Verformen mit Wasser spült.

## Claims

1. Cosmetic composition comprising, in a cosmetically acceptable medium, at least one organic silicon compound of formula (1):
(R₁O)ₓ(R₂)_{y}Si-(A)ₚ-[NR₃-(A')_{p'}]_{q}-[NR'₃-(A")_{p"}]_{q'}-Si-(R'₂)_{y'} (OR'₁)_{x'} (1)
where
R₁, R₂, R'₁ and R'₂ each independently represent a linear or branched, saturated or unsaturated hydrocarbon-based chain optionally containing one or more heteroatoms, optionally interrupted or substituted with one or more groups chosen from ether, ester, amine, amide, carboxyl, hydroxyl and carbonyl groups,
x is an integer ranging from 1 to 3,
y = 3-x,
x' is an integer ranging from 1 to 3,
y' = 3-x',
p = 0 or 1,
p' = 0 or 1,
p" = 0 or 1,
q = 0 or 1,
q' = 0 or 1,
it being understood that at least q or q' is other than zero,
A, A' and A" each independently represent a linear or branched C₁-C₂₀ divalent alkylene radical,
R₃ and R'₃ each independently represent a hydrogen atom or a linear or branched, saturated or unsaturated hydrocarbon-based chain optionally containing one or more heteroatoms, optionally interrupted or substituted with one or more ether, C₁-C₂₀ alcohol ester, amine, carboxyl, alkoxysilane, C₆-C₃₀ aryl, hydroxyl or carbonyl groups, or a heterocyclic or non-heterocyclic aromatic ring, optionally substituted with one or more C₃-C₂₀ alcohol ester, amine, amide, carboxyl, alkoxysilane, hydroxyl, carbonyl or acyl groups,
said cosmetic composition also comprising at least one cosmetic agent chosen from polymers having a molecular weight at least equal to 500, surfactants and fatty substances which are non-silicone.

2. Composition according to Claim 1, **characterized in that** said cosmetically acceptable medium contains at least one C₁-C₈ alcoholic solvent and from 0 to 50% by weight of water relative to the total weight of the composition.

3. Composition according to either one of the preceding claims, **characterized in that**:
R₁ = R'₁,
R₂ = R'₂,
x = x',
y = y',
p = p',
A = A',
q = 1, and
q' = 0.

4. Composition according to any one of the preceding claims, **characterized in that** R₁, R₂, R'₁ and R'₂, which may be identical or different, represent a C₁-C₄ alkyl.

5. Composition according to any one of the preceding claims, **characterized in that** p = p' = 1.

6. Composition according to any one of the preceding claims, **characterized in that** A and A', which may be identical or different, represent a linear C₁-C₄ alkylene.

7. Composition according to any one of the preceding claims, **characterized in that** R₃ is hydrogen.

8. Composition according to any one of Claims 1 to 7, **characterized in that** the compound of formula (1) is water-insoluble and soluble in alcoholic and/or aqueous-alcoholic media.

9. Composition according to any one of the preceding claims, **characterized in that** the organic silicon compound (s) is (are) chosen from:
- bis[3-(triethoxysilyl)propyl]amine, of formula:
(CH₃CH₂O)₃-Si- (CH₂)₃-NH- (CH₂)₃-Si- (OCH₂CH₃)₃,
- bis[trimethoxysilylpropyl]amine, of formula:
(CH₃O)₃-Si- (CH₂)₃-NH- (CH₂)₃-Si- (OCH₃)₃,
- bis[methyldiethoxysilylpropyl]amine, of formula:
(CH₃CH₂O)₂CH₃-Si- (CH₂)₃-NH- (CH₂)₃-Si-CH₃(OCH₂CH₃)₂,
- bis[(3-trimethoxysilyl)propyl]ethylenediamine, of formula:
(CH₃O)₃-Si- (CH₂)₃-NH- (CH₂)₂-NH- (CH₂)₃-Si- (OCH₃)₃, and

10. Cosmetic composition according to Claim 9, **characterized in that** the organic silicon compound is bis[3-(triethoxysilyl)propyl]amine.

11. Composition according to any one of the preceding claims, **characterized in that** the organic silicon compound(s) represent(s) from 0.5% to 50%, preferably from 2% to 25%, by weight relative to the total weight of the composition.

12. Composition according to any one of Claims 1 to 11, **characterized in that** the cosmetic agent(s) chosen from polymers, surfactants and fatty substances which are non-silicone represent(s) from 0.05% to 40%, preferably from 0.1% to 20% and even more preferentially from 0.5% to 10%, by weight of the total weight of the composition.

13. Composition according to any one of Claims 2 to 12, **characterized in that** the C₁-C₈ alcoholic solvent(s) is (are) chosen from alkanols and alkanediols.

14. Composition according to Claim 13, **characterized in that** the solvent(s) is (are) chosen from ethanol, propanol and isopropanol, preferably ethanol.

15. Cosmetic composition according to any one of Claims 2 to 14, **characterized in that** the alcoholic solvent(s) represent(s) from 40% to 95%, preferably from 50% to 90%, by weight relative to the total weight of the composition.

16. Composition according to any one of the preceding claims, **characterized in that** it also comprises at least one additional cosmetic active agent chosen from protein hydrolysates, swelling and penetrating agents, agents for combating hair loss, antidandruff agents, natural or synthetic nonpolymeric thickeners, suspending agents, sequestrants, reducing agents, opacifiers, dyes, sunscreens, vitamins or provitamins, fragrances and preservatives, pH modifiers, and mixtures thereof.

17. Composition according to any one of the preceding claims, **characterized in that** it comprises one or more organic acids.

18. Composition according to Claim 17, **characterized in that** the organic acid(s) is (are) chosen from acetic acid, propanoic acid, butanoic acid, lactic acid, glycolic acid, ascorbic acid, maleic acid, phthalic acid, succinic acid, taurine, tartaric acid, gluconic acid, glucuronic acid and citric acid.

19. Use, for treating the hair, of a cosmetic composition comprising, in a cosmetically acceptable medium, at least one organic silicon compound of formula (1) :
(R₁O)ₓ(R₂)_{y}Si-(A)ₚ-[NR₃-(A')_{p'}]_{q}-[NR'₃-(A")_{p"}]_{q'}-Si-(R'₂)_{y'} (OR'₁)_{x'} (1)
where
R₁, R₂, R'₁ and R'₂ each independently represent a linear or branched, saturated or unsaturated hydrocarbon-based chain optionally containing one or more heteroatoms, optionally interrupted or substituted with one or more groups chosen from ether, ester, amine, amide, carboxyl, hydroxyl and carbonyl groups,
x is an integer ranging from 1 to 3,
y = 3-x,
x' is an integer ranging from 1 to 3,
y' = 3-x',
p = 0 or 1,
p' = 0 or 1,
p" = 0 or 1,
q = 0 or 1,
q' = 0 or 1,
it being understood that at least q or q' is other than zero,
A, A' and A" each independently represent a linear or branched C₁-C₂₀ divalent alkylene radical,
R₃ and R'₃ each independently represent a hydrogen atom or a linear or branched, saturated or unsaturated hydrocarbon-based chain optionally containing one or more heteroatoms, optionally interrupted or substituted with one or more ether, C₁-C₂₀ alcohol ester, amine, carboxyl, alkoxysilane, C₆-C₃₀ aryl, hydroxyl or carbonyl groups, or a heterocyclic or non-heterocyclic aromatic ring, optionally substituted with one or more C₃-C₂₀ alcohol ester, amine, amide, carboxyl, alkoxysilane, hydroxyl, carbonyl or acyl groups.

20. Use according to Claim 19, **characterized in that** said cosmetically acceptable medium contains at least one C₁-C₈ alcoholic solvent and from 0 to 50% by weight of water relative to the total weight of the composition.

21. Use according to either one of Claims 19 and 20, **characterized in that** the compound of formula (1) is water-insoluble and soluble in alcoholic and/or aqueous-alcoholic media.

22. Use according to any one of Claims 19 to 21, **characterized in that** the treatment is hair shaping.

23. Use according to Claims 19 to 22, **characterized in that**:
R₁ = R'₁,
R₂ = R'₂,
x = x',
y = y',
p = p',
A = A',
q = 1, and
q' = 0.

24. Use according to any one of Claims 19 to 23, **characterized in that** R₁, R₂, R'₁ and R'₂, which may be identical or different, represent a C₁-C₄ alkyl.

25. Use according to any one of Claims 19 to 24, **characterized in that** p = p' = 1.

26. Use according to any one of Claims 19 to 25, **characterized in that** A and A', which may be identical or different, represent a linear C₁-C₄ alkylene.

27. Use according to any one of Claims 19 to 26, **characterized in that** R₃ is hydrogen.

28. Use according to any one of Claims 19 to 27, **characterized in that** the organic silicon compound(s) is (are) chosen from:
- bis[3-(triethoxysilyl)propyl]amine, of formula:
(CH₃CH₂O)₃-Si- (CH₂)₃-NH- (CH₂)₃-Si- (OCH₂CH₃)₃,
- bis[trimethoxysilylpropyl]amine, of formula:
(CH₃O)₃-Si- (CH₂)₃-NH- (CH₂)₃-Si- (OCH₃)₃,
- bis[methyldiethoxysilylpropyl]amine, of formula:
(CH₃CH₂O) ₂CH₃-Si- (CH₂)₃-NH- (CH₂)₃-Si-CH₃ (OCH₂CH₃)₂,
- bis[(3-trimethoxysilyl)propyl]ethylenediamine, of formula:
(CH₃O)₃-Si- (CH₂)₃-NH- (CH₂)₂-NH- (CH₂)₃-Si- (OCH₃)₃, and

29. Cosmetic use according to Claim 28, **characterized in that** the organic silicon compound is bis[3-(triethoxysilyl)propyl]amine.

30. Use according to any one of Claims 19 to 29, **characterized in that** the organic silicon compound represents from 0.5% to 25%, preferably from 2% to 15%, by weight relative to the total weight of the composition.

31. Use according to any one of Claims 20 to 30, **characterized in that** the C₁-C₈ alcoholic solvent (s) is (are) chosen from alkanols and alkanediols.

32. Use according to Claim 31, **characterized in that** the solvent(s) is (are) chosen from methanol, ethanol, propanol and isopropanol, preferably ethanol.

33. Use according to any one of Claims 20 to 32, **characterized in that** the alcoholic solvent(s) represent(s) from 40% to 95%, preferably from 50% to 90%, by weight relative to the total weight of the composition.

34. Use according to any one of Claims 19 to 33, **characterized in that** the cosmetic composition contains at least one cosmetic agent chosen from polymers, surfactants and fatty substances which are non-silicone.

35. Use according to any one of Claims 19 to 34, **characterized in that** the composition comprises one or more organic acids.

36. Use according to Claim 35, **characterized in that** the organic acid(s) is (are) chosen from acetic acid, propanoic acid, butanoic acid, lactic acid, glycolic acid, ascorbic acid, maleic acid, phthalic acid, succinic acid, taurine, tartaric acid, gluconic acid, glucuronic acid and citric acid.

37. Process for shaping hair, **characterized in that** it comprises the following steps:
- a composition as defined in any one of Claims 1 to 36 is applied to the hair,
- said composition is left on, preferably for at most 30 minutes and better still between 5 and 15 minutes,
- the hair is shaped.

38. Process according to Claim 37, **characterized in that** the hair is rinsed with water after the leave-on time and before the hair shaping.
